# FASCICULE DE BREVET EUROPEEN

(11) **EP 1 960 387 B1**
(45) Date de publication et mention de la délivrance du brevet: **02.11.2011**
(21) Numéro de dépôt: 06829734.0
(22) Date de dépôt: 22.11.2006
(51) Int. Cl.: C07D 401/12, C07D 409/14, A61K 31/4725, A61K 31/473, A61P 3/00, A61P 25/00

(54) **DERIVES DE ISOQUINOLINE ET BENZO[H]ISOQUINOLINE, LEUR PREPARATION ET LEUR UTILISATION EN THERAPEUTIQUE EN TANT QU ANTAGONISTES DU RECEPTEUR DE L HISTAMINE H3**
ISOCHINOLIN- UND BENZO[H]ISOCHINOLIN-DERIVATE, HERSTELLUNG UND THERAPEUTISCHE VERWENDUNG DAVON ALS ANTAGONISTEN DES HISTAMIN-H3-REZEPTORS
ISOQUINOLINE ET BENZO[H]ISOQUINOLINE DERIVATIVES, PREPARATION AND THERAPEUTIC USE THEREOF AS ANTAGONISTS OF HISTAMINE H3 RECEPTOR

(30) Priorité: 24.11.2005 EP 05111248
(43) Date de publication de la demande: 27.08.2008
(73) Titulaire: SANOFI, 75013 Paris (FR)
(72) Inventeur: DIAZ MARTIN, Juan Antonio, F-75013 Paris (FR); ESCRIBANO ARENALES, Beatriz, F-75013 Paris (FR); JIMENEZ BARGUENO, Maria Dolores, F-75013 Paris (FR)
(74) Mandataire: Le Coupanec, Pascale A.M.P.
(86) Numéro de dépôt international: PCT/EP2006/012246
(87) Numéro de publication internationale: WO 2007/060027

(56) Documents cités:
- EP-A- 1 070 714
- WO-A-02/076925

## Description

La présente invention a pour objet des éthers dérivés de tétrahydroisoquinoline et tétrahydrobenzo[h]isoquinoline, leur procédé de préparation et leurs applications en thérapeutique.

WO 02/076925 décrit des antagonistes de récepteurs H₃ de l'histamine. Ces composés sont pour certains des dérivés éthers d'isoquinolines ou de benzoisoquinolines, éthers sur lesquels sont rattachés des alkyles amines linéaires ou cycliques.

Les inventeurs se sont donnés pour but de parvenir à de nouveaux composés modulant l'activité du récepteur H₃ de l'histamine.

En conséquence la présente invention a pour premier objet les nouveaux composés répondant à la formule I 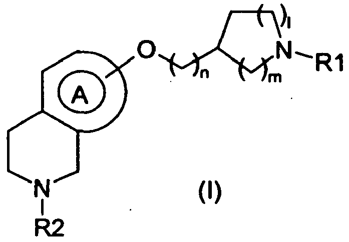 dans laquelle: 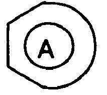 représente un carbocycle insaturé avec doubles liaisons, tel qu'un phényle ou un naphtyle; le carbocycle étant éventuellement substitué par un ou deux substituants choisis, indépendamment l'un de l'autre, parmi un atome d'halogène, un hydroxy, un groupe nitro, cyano, C₁₋₂ perhalogénoalkyle, C₁₋₃ alkyle ou un phényle ;
l peut prendre une valeur de 0 à 4 ;
m peut prendre une valeur de 0 à 3 ;
n peut prendre une valeur de 0 à 6 ;
**-(C)l-, -(C)m-** et **-(C)n-** représentent, indépendamment l'un de l'autre, un groupe - C_{x-z}- alkylidène, éventuellement substitués par 1 à 4 substituants choisis parmi un atome d'halogène, un groupe hydroxy, nitro, cyano, amino, C₁₋₂ perhalogénoalkyle, C₁₋₃ alkyle ou un phényle ; et de plus lorsque l, m et/ou n prend la valeur 0, -(C)₀ représente une liaison ;
**R1** représente un atome d'hydrogène, un groupe C₁₋₃ alkyle, un C₁₋₆ alkylcarbonyle, un C₁₋₆ alkoxycarbonyle, pouvant être substitués, ces groupes C₁₋₃ alkyle, C₁₋₆ alkylcarbonyle et C₁₋₆ alkoxycarbonyle, par un atome d'halogène, un groupe hydroxy, C₁₋₃ alcoxy, nitro, cyano, amino ou un aryle, tel qu'un benzyloxycarbonyl; C₁₋₃ alkyle-aryle tel qu'un benzyle ou phénéthyle, un hétéroaryle monocyclique tel qu'un thiényle, furyle ou pyrrolyle ou un aryle, tel qu'un phényle ou un naphtyle; les groupes aryle et hétéroaryle étant éventuellement substitué par 1 à 4 substituants choisis parmi un atome d'halogène, un groupe hydroxy, nitro, cyano, amino, C₁₋₃ monoalkylamino, C₂₋₆ dialkylamino, C₁₋₃ alkyle, C₁₋₂ perhalogénoalkyle, C₁₋₃ halogénoalkyle, C₁₋₃ alcoxy ou un groupe C₁₋₃ alkylidènedioxy ;
**R2** représente un groupe C₁₋₆ alkyle ou C₃₋₆ cycloalkyle éventuellement substitués par 1 à 4 substituants choisis parmi un atome d'halogène, un groupe hydroxy, nitro, cyano, amino, C₁₋₃ monoalkylamino, C₂₋₆ dialkylamino, C₁₋₂ perhalogénoalkyle, C₁₋₃ halogénoalkyle, C₁₋₃ alcoxy, C₃₋₆ cycloalkyle, un hétéroaryle monocyclique tel qu'un thiényle, furyle ou pyrrolyle, un hétéroaryle bicyclique tel qu'un benzotriazolyle ou un groupe aryle, tel qu'un phényle ou un naphtyle; l'aryle étant éventuellement substitué par 1 à 4 substituants choisis parmi un atome d'halogène, un groupe hydroxy, nitro, cyano, amino, C₁₋₃ monoalkylamino, C₂₋₆ dialkylamino, C₁₋₃ alkyle, C₁₋₂ perhalogénoalkyle, C₁₋₃ halogénoalkyle, C₁₋₃ alcoxy ou un groupe C₁₋₃ alkylidènedioxy.

Dans le cadre de la présente invention, on entend par :
- C_{x-z}, où x et z peuvent prendre les valeurs de 0 à 6, une chaîne carbonée pouvant avoir de x à z atomes de carbone, toutefois lorsque x prend la valeur 0, C₀ représente une liaison; par exemple C₁₋₆ indique une chaîne carbonée pouvant avoir de 1 à 6 atomes de carbone; C₀₋₆ indique une liaison ou une chaîne carbonée pouvant avoir de 1 à 6 atomes de carbone;
- alkyle, un groupe aliphatique saturé linéaire ou ramifié; par exemple, un groupe C₁₋₆ alkyle représente une chaîne carbonée de 1 à 6 atomes de carbone saturée, linéaire ou ramifiée, plus particulièrement un radical méthyle, éthyle, propyle, isopropyle, butyle, isobutyle, tertbutyle, etc. ;
- le terme C_{x-y} alkylidène désignant un groupe C_{x-y} alkyle, linéaire ou ramifié, divalent; le terme C₂₋₈ alkenylidène désignant un groupe C_{x-y} alkyle, insaturé, linéaire ou ramifié, divalent ;
- C_{x-y} alcoxy, un groupe alkyloxy à chaîne aliphatique saturée, linéaire ou ramifiée, comportant x à y atomes de carbone;
- atome d'halogène, un fluor, un chlore, un brome ou un iode ;
- C₁₋₃ monoalkylamino, un amino mono substitué par un groupe C₁₋₃ alkyle ;
- C₂₋₆ dialkylamino, un amino di-substitué par deux groupes C₁₋₃ alkyle, identiques ou différents ;
- C₁₋₂ perhalogénoalkyle, un groupe C₁₋₂ alkyle dans lequel tous les atomes d'hydrogène sont substitués par des atomes d'halogène ;
- C₁₋₃ halogénoalkyle, un groupe C₁₋₃ alkyle dans lequel au moins un atome d'hydrogène sont substitués par un atome d'halogène.

Les composés de formule I peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule générale I peuvent se présenter sous forme de bases libres ou de sels d'addition à des acides, qui font également partie de l'invention. Ces sels, selon la présente invention, comprennent ceux avec des acides pharmaceutiquement acceptables mais aussi ceux avec des acides minéraux ou organiques qui permettent une séparation ou une cristallisation convenable des composés de formule I. Ces sels peuvent être préparés, selon des méthodes connues de l'homme du métier, par exemple, par réaction du composé de formule I sous forme de base avec l'acide dans un solvant approprié, tel qu'une solution alcoolique ou un solvant organique, puis séparation du milieu qui le contient par évaporation du solvant ou par filtration.

Les composés de formule I peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

La présente invention a également pour objet les composés choisis parmi les sous-groupes suivants, considérés seuls ou en combinaison, dans lesquels: 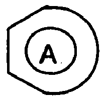 représente un carbocycle insaturé, tel qu'un phényle ou un naphtyle; le carbocycle étant éventuellement substitué par 1 ou 2 substituants choisis, indépendamment l'un de l'autre, parmi un atome d'halogène, un groupe hydroxy, nitro, cyano, C₁₋₂ perhalogénoalkyle ou C₁₋₃ alkyle;
I peut prendre une valeur de 1, 2 ou 3;
m peut prendre une valeur de 0, 1 ou 2;
n peut prendre une valeur de 0, 1, 2 ou 3 ;
**-(C)l-** et **-(C)m-** forment ensemble, avec le groupe -NR1-, un aminocycle, lié par un carbone au groupe **-O-(C)n-** tel qu'azétidine, pyrrolidine, pipéridine ou azépine et/ou
**-(C)n-** représente un groupe -C₀₋₃ -alkylidène, éventuellement substitué par 1 à 4 substituants choisis parmi un atome d'halogène, un groupe hydroxy, nitro, cyano, amino, C₁₋₂ perhalogénoalkyle; toutefois lorsque n prend la valeur 0, -(C)₀- représente une liaison ;
**R1** représente un atome d'hydrogène, un groupe C₁₋₃ alkyle, un C₁₋₄ alkylcarbonyle, un C₁₋₆ alkoxycarbonyle; C₁₋₃ alkylaryle tel qu'un benzyle, un hétéroaryle tel qu'un thiényle ou furyle, un groupe aryle, tel qu'un phényle ou un naphtyle; les groupes aryle et hétéroaryle étant éventuellement substitué par 1 à 4 substituants choisis parmi un atome d'halogène, un groupe hydroxy, cyano, amino, C₁₋₃ monoalkylamino, C₁₋₃ alkyle, C₁₋₂ perhalogénoalkyle, C₁₋₃ halogénoalkyle, C₁₋₃ alcoxy ou C₁₋₃ alkylidènedioxy .

Plus particulièrement, lorsque l'aminocycle dont fait partie -(C)I-, -(C)m- et -NR1- et lequel est lié par un carbone au groupe -O-(C)n-, est choisi parmi les groupes suivants: 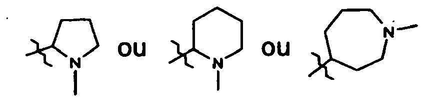 **R2** représente un groupe C₁₋₄ alkyle ou C₅₋₆ cycloalkyle éventuellement substitués par 1 à 4 substituants choisis parmi un phényle, un hétéroaryle monocyclique tel qu'un thiényle, un hétéroaryle bicyclique tel qu'un benzotriazolyle, un groupe C₃₋₆ cycloalkyle, C₁₋₂ perhalogénoalkyle, C₁₋₃ halogénoalkyle ou C₁₋₃ alcoxy ; le phényle et l'hétéroaryle étant éventuellement substitués par 1 à 4 substituants choisis parmi un atome d'halogène, un groupe hydroxy, nitro, cyano, amino, C₁₋₃ monoalkylamino, C₂₋₆ dialkylamino, C₁₋₃ alkyle, C₁₋₂ perhalogénoalkyle, C₁₋₃ halogénoalkyle, C₁₋₃ alcoxy ou un groupe C₁₋₃ alkylidènedioxy.

Un autre objet de la présente invention, concerne les composés suivants, et leurs sels pharmaceutiquement acceptables:
Composé 1 : 7-{2-[1-méthylpipéridin-2-yl]éthoxy}-2-propyl-1,2,3,4-tétrahydroisoquinoline;
Composé 2 : 2-isobutyl-7-[2-(1-méthylpyrrolidin-2-yl)éthoxy]-1,2,3,4-tétrahydroisoquinoline;
Composé 3: 2-(3-méthylbutyl)-7-[2-(1-méthylpyrrolidin-2-yl)éthoxy]-1,2,3,4-tétrahydroisoquinoline;
Composé 4: 7-[(1-méthylazepan-4-yl)oxy]-2-(3-méthylbutyl)-1,2,3,4-tétrahydroisoquinoline
Composé 5 : (2-cyclohéxylméthyl)-7-[2-(1-méthylpyrrolidin-2-yl)éthoxy]-1,2,3,4-tétrahydroisoquinoline;
Composé 6 : (2-cyclohéxylméthyl)-7-{2-[(2*R*)-1-méthylpyrrolidin-2-yl]éthoxy}-1,2,3,4-tétrahydroisoquinoline;
Composé 7: (2-cyclohéxylméthyl)-7-{2-[(2*S*)-1-méthylpyrrolidin-2-yl]éthoxy}-1,2,3,4-tétrahydroisoquinoline;
Composé 8: (2-cyclohéxylméthyl)-7-[(1-méthylazepan-4-yl)oxy]-1,2,3,4-tétrahydroisoquinoline;
Composé 9: (2-cyclohéxylméthyl)-7-[2-(1-méthylpipéridin-2-yl)éthoxy]-1,2,3,4-tétrahydroisoquinoline;
Composé 10: 2-benzyl)-7-[2-(1-méthylpyrrolidin-2-yl)éthoxy]-1,2,3,4-tétrahydroisoquinoline;
Composé 11 : 2-benzyl-7-[(1-méthylazepan-4-yl)oxy]-1,2,3,4-tétrahydroisoquinoline;
Composé 12: 7-[(1-méthylazepan-4-yl)oxy]-2-(2-thiénylméthyl)-1,2,3,4-tétrahydroisoquinoline ;
Composé 13: (2-cyclohéxylméthyl)-8-[2-(1-méthylpyrrolidin-2-yl)éthoxy]-1,2,3,4-tétrahydrobenzo[*h*]isoquinoline;
Composé 14 : (2-cyclohéxylméthyl)-8-{2-[(2*R*)-1-méthylpyrrolidin-2-yl]éthoxy}-1,2,3,4-tétrahydrobenzo[*h*]isoquinoline;
Composé 15 : (2-cyclohéxylméthyl)-8-{2-[(2*S*)-1-méthylpyrrolidin-2-yl]éthoxy}-1,2,3,4-tétrahydrobenzo[*h*]isoquinoline;
Composé 16: 2-(cyclohéxylméthyl)-8-[(1-méthylazepan-4-yl)oxy]-1,2,3,4-tetrahydrobenzo[*h*]isoquinoline ;
Composé 17: (2-cyclohéxylméthyl)-8-[2-(1-méthylpipéridin-2-yl)éthoxy]-1,2,3,4-tétrahydrobenzo[*h*]isoquinoline;
Composé 20 : 2-butyl-7-[(1-méthylazepan-4-yl)oxy]-1,2,3,4-tétrahydroisoquinoline; Composé 21: 2-butyl-7-[2-(1-méthylpyrrolidin-2-yl)éthoxy]-1,2,3,4-tétrahydroisoquinoline ;
Composé 22: 7-[(1-méthylazepan-4-yl)oxy]-2 propyl)-1,2,3,4-tétrahydroisoquinoline; Composé 23: 7-[2-(1-méthylpyrrolidin-2-yl)éthoxy]- 2-propyl-1,2,3,4-tétrahydroisoquinoline.

D'autre part, dans le cadre de la présente invention, on entend par groupe protecteur Pg, un groupe qui permet d'une part de protéger une fonction réactive telle qu'un hydroxy, ou une amine pendant une synthèse et d'autre part de régénérer la fonction réactive intacte en fin de synthèse. Des exemples de groupes protecteurs ainsi que les méthodes de protection et déprotection sont données dans «Protective groups in Organic Synthesis 3th Ed.», Greene et Wuts (John Wiley & Sons, Inc., New York, 1999).

La présente invention a pour second objet un procédé de préparation des composés de formule I selon l'invention.

Ainsi, les composés de formule I peuvent être préparés selon le procédé représenté dans le schéma 1.

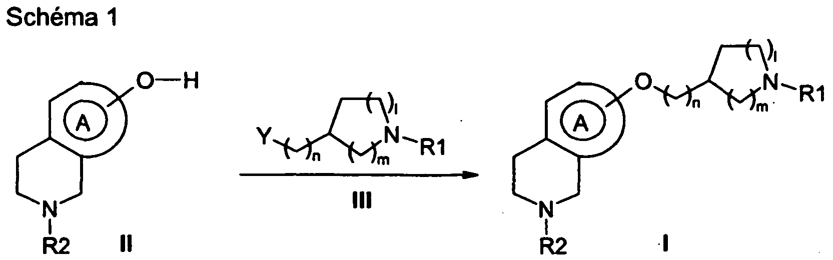

Selon le procédé du schéma 1, les composés de formule I, dans laquelle R1, R2, I, m, n et le cycle A sont tels que défini dans la formule I, sont préparés par substitution nucléophile, en faisant réagir un phénol de formule II, dans laquelle R2 et le cycle A sont tels que défini dans la formule I, avec une amine de formule III, dans laquelle R1, l, m et n sont définis comme dans la formule I et Y représente un atome d'halogène, tel que par exemple un chlore, iode ou brome, ou représente un «pseudo-halogène» tel qu'un mesylate, triflate, tosylate, brosylate ou nosylate. La réaction peut être réalisée dans un solvant protique ou aprotique, tel que l'eau, le méthanol, l'acétone, la butanone, l'acétate d'éthyle, le toluène, la N,N-dimethylformamide, l'acétonitrile ou un mélange de ces solvants, à une température comprise entre 0 et 110°C, en présence d'une base telle que, par exemple, l'hydroxyde de sodium ou de potassium, le carbonate de sodium ou de potassium, la triéthylamine ou la diisopropyléthylamine, pour donner le composé de formule I. Dans le cas des mélanges de solvants non miscibles on peut utiliser un catalyseur de transfert de phase, tel qu'un sel d'ammonium ou de phosphonium, de préférence le bromure de tétrabutylammonium ou le chlorure de tétraéthylammonium, dans un mélange du toluène et d'eau, à une température comprise entre 20 et 110°C. Si nécessaire, les composés de formule II et III peuvent être préalablement protégés avant réaction selon des méthodes connues de l'homme du métier. Le composé de formule I est ensuite éventuellement déprotégé selon des conditions connues de l'homme du métier.

Alternativement, les composés de formule I peuvent être préparés par une réaction de type Mitsunobu. Selon cette alternative, on fait réagir un phénol de formule II, dans laquelle R2 et le cycle A sont tels que défini dans la formule I, avec une amine de formule III, dans laquelle R1, I, m et n sont définis comme dans la formule I, mais Y représente un groupe hydroxy, obtenue selon des méthodes connues de l'homme du métier. La réaction peut être réalisée de façon classique en présence de réactifs de Mitsunobu tel qu'un azodérivé, par exemple, le diéthylazodicarboxylate, le diisopropylazodicarboxylate, le diterbutylazodicarboxylate, la 1,1'-(azodicarbonyl)dipipéridine ou la *N*,*N*,*N'*,*N*'-tetraméthylazodicarboxamide et une phosphine, par exemple, la triphénylphosphine ou la tributylphosphine. La réaction peut être réalisée dans un solvant aprotique, tel que le tétrahydrofurane ou le dioxane ou un mélange de ces solvants, à une température comprise entre 0 et 100°C, pour donner le composé de formule I. Le composé de formule I, si les réactifs ont dû être préalablement protégés avant réaction, est déprotégé selon des conditions connues de l'homme du métier.

Les composés de départ protégés (formule VI) ou non protégés (formule II), peuvent être préparés selon le schéma 2 ou peuvent être synthétisés par des méthodes classiques connues de l'homme du métier, tel que le Journal of Medicinal Chemistry 40, 3997-4005 (1997) ou Tetrahedron Assymmetry 12, 2427-2434 (2001).

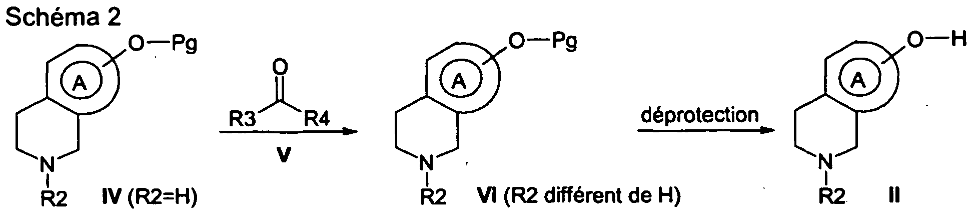

Selon le procédé du schéma 2, les composés de formule II, dans laquelle R2 est tel que défini dans la formule I, mais différent d'un atome d'hydrogène, sont préparés par amino-réduction, en faisant réagir une amine secondaire de formule IV, dans laquelle R2 représente H, avec un aldéhyde ou une cétone de formule V, où R3 et R4, après réaction forment ensemble R2 tel que défini dans la formule I et différent d'hydrogène. Les composés de formule IV, où R2 représente un atome d'hydrogène, peuvent être obtenus selon des méthodes classiques connues de l'homme du métier, tel que le Journal of Medicinal Chemistry 40, 3997-4005 (1997). Les composés de formule II peuvent être ensuite obtenus à partir des composés de formule VI, qui sont déprotégés selon des conditions connues de l'homme du métier. Par exemple, les composés de formule VI, lorsque Pg est un groupe méthyle, peuvent être déprotégés en présence d'un acide tel que l'acide bromhydrique dans un solvant protique, tel que l'eau ou l'acide acétique ou un mélange de ces solvants, à une température comprise entre 0 et 100°C, dans la présence, ou non, d'un catalyseur de transfert de phase, tel qu'un sel d'ammonium ou de phosphonium, pour donner le phénol de formule II. Des illustrations du procédé sont données dans les exemples.

Alternativement, selon le procédé du schéma 3, les composés de formule II dans laquelle R2 est tel que défini dans la formule I, mais différent d'un atome d'hydrogène, peuvent être préparés en faisant réagir le composé protégé de formule VI, dans laquelle R2 représente un groupe benzotriazolylméthyl, obtenu, par exemple, selon le procédé décrit dans Tetrahedron Assymmetry 12, 2427-2434 (2001), avec un agent d'alkylation tel qu'un réactif de Grignard approprié.

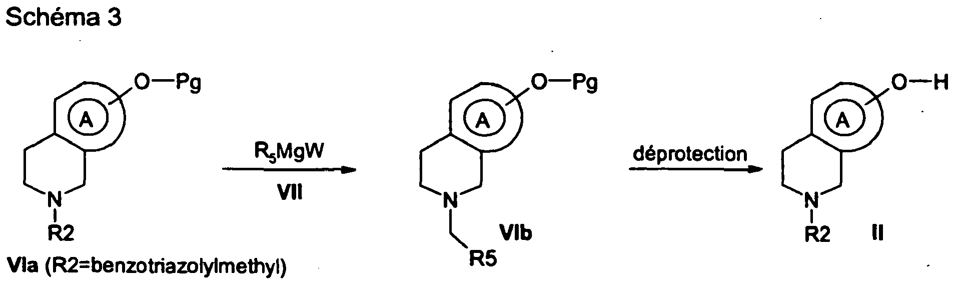

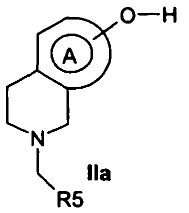

Selon cette alternative, les composés de formule II, dans laquelle R2 est tel que défini dans la formule I, sont préparés par substitution nucleophilique, en faisant réagir un composé de formule Via, dans laquelle R2 représente un groupe benzotriazolylmethyl, avec un réactif de Grignard de formule VII, où W représente un atome d'halogène, tel que par exemple un chlore, iode ou brome et R5 représente un groupe C₁₋₅ alkyle, C₁₋₂ perhalogénoalkyle, C₁₋₃ halogénoalkyle, C₃₋₆ cycloalkyle, un hétéroaryle monocyclique tel qu'un thiényle ou furyle ou un groupe aryle, tel qu'un phényle ou un naphtyle ; après réaction, on forme le composé de formule II où R2 est tel que défini dans la formule I et différent d'hydrogène. La réaction peut être réalisée dans un solvant aprotique, tel que le éther diéthylique, le tétrahydrofurane ou le dioxane ou un mélange de ces solvants, à une température comprise entre -70 et 100°C. pour donner le composé de formule VI. Les composés de formule II peuvent être ensuite obtenus à partir des composés de formule VI, qui sont déprotégés selon des conditions connues de l'homme du métier. Par exemple, les composés de formule VI, lorsque Pg est un groupe méthyle, peuvent être déprotégés en présence d'un acide tel que l'acide bromhydrique dans un solvant protique, tel que l'eau ou l'acide acétique ou un mélange de ces solvants, à une température comprise entre 0 et 100°C, dans la présence, ou non, d'un catalyseur de transfert de phase, tel qu'un sel d'ammonium ou de phosphonium, pour donner le phénol de formule II. Des illustrations du procédé sont données dans les exemples.

Les composés de départ II et les amines de formule III sont directement disponibles dans le commerce ou peuvent être synthétisés par des méthodes décrites comme préalablement, par des méthodes classiques connues de l'homme du métier ou sont connus dans la littérature.

Selon un autre de ses aspects, l'invention a pour objet des médicaments qui comprennent un composé de formule I ou un sel d'addition de ce dernier à un acide pharmaceutiquement acceptable ou encore un hydrate ou un solvat du composé de formule I.

Ces composés de la présente invention trouvent leur emploi en thérapeutique, notamment le traitement des troubles améliorés par modulation du récepteur H₃ de l'histamine, et dans le traitement des pathologies dans lesquelles un antagoniste du récepteur de l'histamine du type H₃ apporte un bénéfice thérapeutique. Notamment de telles pathologies sont l'obésité et le diabète.

Ces composés avec des propriétés comme antagoniste et agoniste inverse du récepteur H3 de l'histamine dans le traitement des maladies du système nerveux central.

Egalement, ces composés peuvent être employés dans le traitement des maladies du système nerveux central telles que troubles de la vigilance et du sommeil, la narcolepsie, la maladie d'Alzheimer et autres démences, la maladie de Parkinson, les troubles de l'attention chez l'enfant hyperkinétique, les troubles de la mémoire et de l'apprentissage, l'épilepsie, la schizophrénie, les troubles cognitifs modérés, la dépression et l'anxiété. Les états de dépression et d'anxiété comprennent, par exemple, les anxiétés de type anticipatoire (avant intervention chirurgicale, avant traitement dentaire, etc), l'anxiété causée par la dépendance ou le sevrage d'alcool, de drogue, la manie, les désordres affectifs saisonniers, les migraines et les nausées. Ils peuvent être aussi utilisés dans le traitement des dysfonctionnements sexuels, des vertiges et du mal des voyages.

L'utilisation des composés selon l'invention pour la préparation d'un médicament destiné à traiter les pathologies ci-dessus mentionnées fait partie intégrante de l'invention.

Selon un autre de ses aspects, la présente invention concerne des compositions pharmaceutiques comprenant, en tant que principe actif, au moins un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un ou plusieurs excipients pharmaceutiquement acceptables. Les dits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule I ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

Afin d'obtenir l'effet prophylactique ou thérapeutique désiré, la dose de principe actif peut varier entre 0,1 µg et 50 mg par kg de poids du corps et par jour. Chaque dose unitaire peut contenir de 0,1 à 1000 mg, de préférence de 1 à 500 mg, de principe actif en combinaison avec un.excipient pharmaceutique. Cette dose unitaire peut être administrée 1 à 5 fois par jour de façon à administrer un dosage journalier de 0,5 à 5000 mg, de préférence de 1 à 2500mg.

II peut y avoir des cas particuliers où des dosages plus élevés ou plus faibles sont appropriés. De tels dosages appartiennent également à l'invention. Selon la pratique habituelle, le dosage approprié à chaque patient est déterminé par le médecin selon le mode d'administration, le poids et la réponse dudit patient.

A titre d'exemple, une forme unitaire d'administration d'un composé selon l'invention:

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

La présente invention selon un autre de ses aspects, concerne également une méthode de traitement des pathologies ci-dessus indiquées qui comprend l'administration, à un patient, d'une dose efficace d'un composé selon l'invention ou un des ses sels pharmaceutiquement acceptables ou hydrates ou solvats.

Les exemples suivants illustrent les procédés et techniques appropriés pour la préparation de cette invention, sans toutefois limiter l'étendue de sa portée.

### Exemple 1- Oxalate (2:1) de (2-cyclohéxylméthyl)-7-[2-(1-méthylpyrrolidin-2-yl)éthoxy]-1,2,3,4-tétrahydroisoquinoline

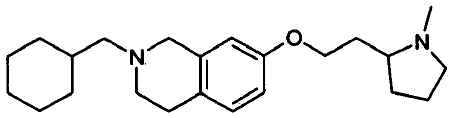

### 1.1- 2-(cyclohéxylméthyl]-7-méthoxy-1,2,3,4-tétrahydroisoquinoline

A une solution de 10,41 g (0,035 mole) de 2-(1*H*-1,2,3-benzotriazol-1-ylméthyl)-7-méthoxy-1,2,3,4-tétrahydroisoquinoline dans 150 ml de tétrahydrofurane, refroidie à - 40°C, sont ajoutés 35 ml (0,070 mole) d'une solution 2N de cyclohéxyl magnesium chloride dans le tétrahydrofurane. L'agitation est maintenue pendant deux heures à 40°C et puis le mélange est laissé à reposer durant la nuit à température ambiante. Une solution aqueuse d'une solution 2N d'hydroxide de sodium (50 ml) est ajoutée. La phase aqueuse est extraite 3 fois avec 20ml d'éther éthylique et les phases organiques sont séchées et évaporées à sec sous vide. On obtient 8,20 g d'huile utilisée sans purification additionnelle.
Rdt: 90%
PF= huile

### 1.2- Bromhydrate de 2-(cyclohéxylméthyl]-1,2,3,4-tétrahydroisoquinolin-7-ol

Une solution de 8,20 g (0,032 mole) du 2-(cyclohéxylméthyl]-7-méthoxy-1,2,3,4-tétrahydroisoquinoline dans 80ml de bromure d'hydrogène aqueux (48%) est chauffée durant six heures à 120°C. Le mélange est refroidi, concentré a sec et le residue est traité avec 60 ml d'un mélange d'éthanol/ éther éthylique . Le solide qui s'est formé, est filtré, lavé avec de l'éther éthylique et séché. On obtient 9,70 g du produit désiré comme un solide blanc pur.
Rdt: 94%
PF= 210-214°C

### 1.3- Oxalate (2:1) de (2-cyclohéxylméthyl)-7-[2(1-méthylpyrrolidin-2-yl)éthoxy]-1,2,3,4-tétrahydroisoquinoline

A un mélange de 5,00 g (0,020 mole) du composé obtenu précédemment au 1.2, 11,2 g (0,0061 mole) de 2-(2-chloroéthyl)-1-méthylpyrroline et 0,41 g (0,002 mole) du chlorure de triéthylammonium dans 125 ml de toluène, est ajouté un mélange de 9,7 g (0,244 mole) d'hydroxide de sodium dans 125 ml d'eau. On chauffe la réaction a reflux durant huit heures. Les phases sont séparées et la phase aqueuse est extraite 2 fois avec 20ml de toluène. Les phases organiques sont séchées et puis évaporées à sec. On obtient 7g (97%) d'une huile brute obtenue qui est purifiée par chromatographie sur colonne de gel de silice, avec un mélange de dichlorométhane/méthanol (98:2) employé comme éluant. Le produit désiré (0,70 g; 10%) est obtenu sous forme d'une huile avec Rf le plus grand.

¹H-NMR (CDCl3) δ(ppm): 7,1(1H, d), 6.7(1H, d), 6.5(1H, s), 4.0(2H, m), 3,5 (2H, s), 3.0 (1H, m), 2,7 (2H, m), 2,6 (2H, m), 2,4 (3H, s), 2,2 (2H,d),2.1 (2H,m) 2.0(1 H,m), 1.7 (11H, m), 1,1(3H, m), 0,9 (2H, m).

L'huile précédente (0,65g ; 0,002 mole) est dissoute dans 10 ml d'éthanol, puis 0,36 g (0,004 mole) d'acide oxalique, dissous dans 10 ml d'éthanol, est ajouté. Le précipité est filtré et lavé avec de l'éthanol froid. On obtient 0,46 g du produit désiré comme un solide blanc.
Rdt: 47%
PF= 78-98°C

### Exemple 2- Oxalate (2:1) de (2-cyclohéxylméthyl)-7-[(1-méthylazepan-4-yl)oxy]-1,2,3,4-tétrahydroisoquinoline

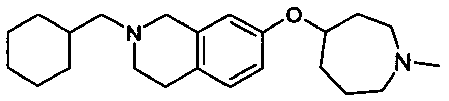

Selon le procédé décrit précédemment au 1.3, on obtient une huile de Rf le plus petit obtenu (1,30 g ; 0,002 mole) qui correspond à la structure du (2-cyclohéxylméthyl)-7-[(1-méthylazepan-4-yl)oxy]-1,2,3,4-tétrahydroisoquinoline.

**¹H-NMR (CDCl3) δ(ppm):** 7,1(1H, d), 6.7(1H, d), 6.5(1H, s), 4.5(1H, m), 3,5 (2H, s), 2,7 (2H, m), 2,6-2.4 (5H, m), 2,3 ( 3H, s), 2,2 ( 2H,d), 2.0 (2H,m), 1.7 (11H, m), 1,1(3H, m), 0,9 (2H, m).

L'huile est dissoute dans 12 ml d'éthanol, puis 0,24 g (0,003 mole) d'acide oxalique, dissous dans 12 ml d'éthanol, est ajouté. Le précipité est filtré et lavé avec de l'éthanol froid. On obtient 0,46 g du produit désiré comme un solide blanc.
Rdt: 86%
PF= 110-112°C

### Exemple 3- Oxalate (2:1) 2-cyclohéxylméthyl)-8-[2-(1-méthylpyrrolidin-2-yl)éthoxy]-1,2,3,4-tétrahydrobenzo[h]isoquinoline

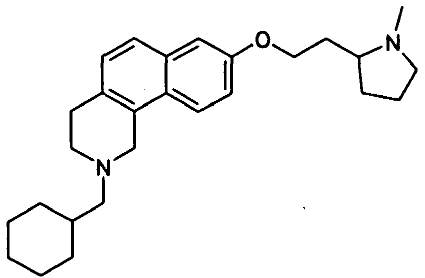

### 3.1- 2-(cyclohéxylméthyl]-8-méthoxy-1,2,3,4-tétrahydroisobenzo[h]quinoline

A une solution de 3 g (0,014 mole) de 8-méthoxy-1,2,3,4-tétrahydroisobenzo[h]quinoline et 1.6g (0,014mole) de cyclohexane-carboxaldéhyde dans 70 ml de méthanol, on ajoute 0,32 g (0,0003 mole) de palladium sur charbon à 10%. La solution est hydrogénée pendant 24 heures dans un hydrogénateur de Paar à une pression de 45 Psi. Le catalyseur est éliminé par filtration et la solution filtrée est évaporée à sec. On obtient 4 g du produit désiré comme une huile.
Rdt: 93%
PF : huile

### 3.2- Bromhydrate de 2-(cyclohéxylméthyl]-1,2,3,4-tétrahydrobenzo[h]isoquinolin-7-ol

Une solution de 2 g (0,006 mole) du 2-(cyclohéxylméthyl]-8-méthoxy-1,2,3,4-tétrahydroisobenzo[h]quinoline dans 30 ml de bromure d'hydrogène aqueux (48%) est chauffée durant six heures à 120°C. Le mélange est refroidi, concentré a sec et le redue est traité avec 20 ml d'un mélange d'éthanol/ éther éthylique . Le solide qui s'est formé, est filtré, lavé avec de l'éther éthylique et séché. On obtient 2.3g du produit désiré comme un solide blanc pur.
Rdt: 96%
PF= 270-276°C

### 3.3- Oxalate (2:1) 2-cyclohéxylméthyl)-8-[2-(1-méthylpyrrolidin-2-yl)éthoxyl-1,2,3,4-tétrahydrobenzo[h]isoquinoline

A un mélange de 2,00 g (0,005 mole) du composé obtenu précédemment au 3.2, 2.4 g (0,014 mole) de 2-(2-chloroéthyl)-1-méthylpyrroline et 0.11 g (0,0006 mole) du chlorure de triéthylammonium dans 75 ml de toluène, est ajouté un mélange de 2.5 g (0,064 mole) d'hydroxyde de sodium dans 75ml d'eau. On chauffe la réaction a reflux durant huit heures. Les phases sont séparées et la phase aqueuse est extraite 2 fois avec 20ml de toluène. Les phases organiques sont séchées et puis évaporées à sec. On obtient 3 g (>100%) d'une huile brute obtenue qui est purifiée par chromatographie sur colonne de gel de silice, avec un mélange de dichlorométhane/méthanol (98:2)employé comme éluant. Le produit désiré (0,70 g; 10%) est obtenu sous forme d'une huile avec Rf le plus grand.

**¹H-NMR (CDCl3) δ(ppm):),** 7,7 (1H, d), 7,5 (1H, d), 7.1-7.0 (3H,m), 4.2(2H, m), 4.0 (2H, s), 3.0( 1H, m), 2,9 (2H, m), 2.7(2H,m), 2,4 ( 2H, d), 2,3 ( 3H,s), 2.2-2.0 (3H,m), 1.7 (11H, m), 1,1 (3H, m), 0,9 (2H, m).

L'huile précédente (0, 5g ; 0,001 mole) est dissoute dans 10 ml d'éthanol, puis 0,24 g (0,003 mole) d'acide oxalique, dissous dans 10 ml d'éthanol, est ajouté. Le précipité est filtré et lavé avec de l'éther éthylique froid pour obtenir 0,50 g du produit désiré comme un solide blanc.
Rdt: 70%
PF= 127-135°C

### Exemple 4 - Oxalate (2:1) 2-(cyclohéxylméthyl)-8-[(1-méthylazepan-4-yl)oxy]-1,2,3,4-tetrahydrobenzo[h]isoquinoline

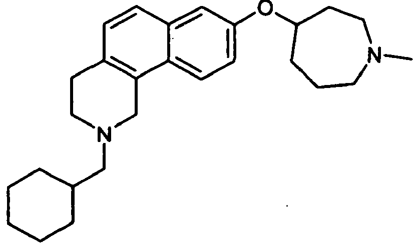

Selon le procédé décrit précédemment au 3.3, on obtient une huile de Rf le plus petit obtenu (0.67 g ; 0,002 mole) qui correspond à la structure du (2-cyclohéxylméthyl)-7-[(1-méthylazepan-4-yl)oxy]-1,2,3,4-tétrahydroisoquinoline

**¹H-NMR (CDCl3) δ(ppm):),** 7,8 (1H, d), 7,6 (1H, d), 7.2-7.0 (3H,m), 4.7 (1H, m), 4.0 (2H, s), 3.0(2H, m), 2,9 (2H, m), 2.7(2H,m), 2,4 (2H, d), 2,3 (3H,s), 2.3-1.7 (14H,m), 1,1(3H, m), 0,9 (2H, m).

L'huile est dissoute dans 10 ml d'éthanol, puis 0,37 g (0,004 mole) d'acide oxalique, dissous dans 10 ml d'éthanol, est ajouté. Le précipité est filtré et lavé avec de l'éther éthylique froid pour obtenir 0,25g du produit désiré comme un solide blanc.
Rdt: 31 %
PF= 77-101°C

### Exemple 5 - Chlorhydrate (2:1) de (2-cyclohéxylméthyl)-7-{2-[(2S)-1-méthylpyrrolidin-2-yl]éthoxy}-1,2,3,4-tétrahydroisoquinoline

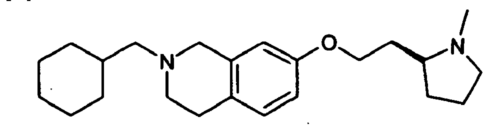

A un mélange de 7,3 g (0,030 mole) du 2-(cyclohéxylméthyl]-1,2,3,4-tétrahydroisoquinolin-7-ol obtenue précédemment au 1.2, 3,5 g (0,027 mole) de (*S*)-2-(2-hydroxyéthyl)1-méthylpyrroline et 9,2 g (0,036mole) de la triphénylphosphine dans 150 ml de tétrahydrofurane, refroidie à -5°C, sons ajoutés 6,6 g (0,032 mole) de diisopropylazodicarboxylate L'agitation est tenue pendant la nuit à température ambiante. La solution est évaporée à sec et on obtient une huile brute qui est purifiée par chromatographie sur colonne de gel de silice, avec un mélange de dichlorométhane/méthanol (95 :5), employé comme éluant. Le produit désiré (1.4g ; 15%) est obtenu sous forme d'une huile.

**¹H-NMR (CDCl3) δ(ppm):** 7,1(1H, d), 6.7(1H, d), 6.5(1H, s), 4.0(2H, m), 3,5 (2H, s), 3.0 (1H, m), 2,7 (2H, m), 2,6 (2H, m), 2,4 (3H, s), 2,2 (2H,d),2.1(2H,m) 2.0(1 H,m), 1.7 (11H, m), 1,1(3H, m), 0,9 (2H, m).

L'huile est dissoute dans 25 ml d'isopropanol, puis d'isopropanol saturé de HCl. Le précipité est filtré et lavé avec de 1 ml d'isopropanol froid. On obtient 1g du produit désiré comme un solide blanc.
Rdt: 59%
PF= 238-241 °C

### Example 6- Chlorhydrate (2:1) 2-cyclohéxylméthyl)-8-[(2S)-(1-méthylpyrrolidin-2-yl)éthoxy]-1,2,3,4-tétrahydrobenzo[h]isoquinoline

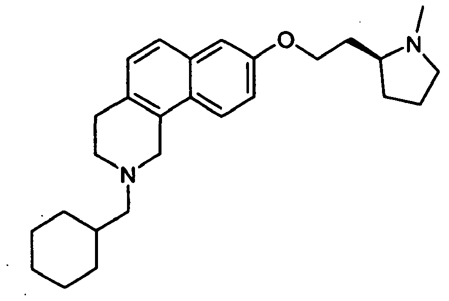

A un mélange de 2,2 g (0,007 mole) du 2-(cyclohéxylméthyl]-1,2,3,4-tétrahydrobenzo[h]isoquinolin-7-ol composé obtenue précédemment au 3.2, 0,95 g (0,007 mole) de (*S*)-2-(2-hydroxyéthyl)1-méthylpyrroline et 2,14 g (0,008mole) de la triphénylphosphine dans 150 ml de tétrahydrofurane, refroidie à -5°C, sons ajoutés 1,88g (0,008 mole) de diisopropylazodicarboxylate L'agitation est tenue pendant la nuit à température ambiante. La solution est évaporée à sec et on obtient une huile brute qui est purifiée par chromatographie sur colonne de gel de silice, avec un mélange de dichlorométhane/méthanol (95 :5), employé comme éluant. Le produit désiré (1,1g ; 36%) est obtenu sous forme d'une huile.

**¹H-NMR (CDCl3) δ(ppm):**), 7,7 (1H, d), 7,5 (1H, d), 7.1-7.0 (3H,m), 4.2(2H, m), 4.0 (2H, s), 3.0( 1H, m), 2,9 (2H, m), 2.7(2H,m), 2,4 (2H, d), 2,3 (3H,s), 2.2-2.0 (3H,m), 1.7 (11H, m), 1,1 (3H, m), 0,9 (2H, m).

L'huile est dissoute dans 15 ml d'isopropanol, puis d'isopropanol saturé de HCl. Le précipité est filtré et lavé avec de 1 ml d'isopropanol froid. On obtient 0.8 g du produit désiré comme un solide blanc.
Rdt: 62%
PF= 260°C

Le tableau ci-après illustre les structures chimiques et les propriétés physiques de quelques composés selon l'invention. Les micro-analyses élémentaires et les spectres RMN, IR ou de masse confirment les structures des composés obtenus.

Dans le tableau, pour les composés de formule I « P.F. » correspond au point de fusion, et <Config> indique la configuration stéréochimique, à savoir (R), (S), ou un mélange racémique (R,S), de l'atome de carbone indiqué par l'astérisque (*).
**Tableau**

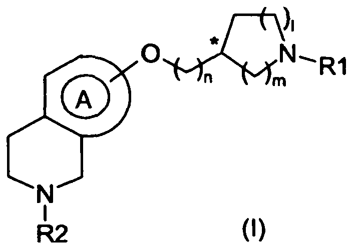

| N° | R2 | R1 | A | n | m | l | Config. | P.F. (°C) | sel |
|---|---|---|---|---|---|---|---|---|---|
| 1. | 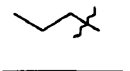 | CH₃ | 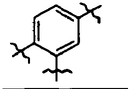 | 2 | 0 | 3 | (*R*,*S*) | 186-190 | Chlorhydrate |
| 2. | 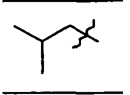 | CH₃ | 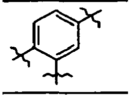 | 2 | 0 | 2 | (*R*,*S*) | 77-80 | Tartrate |
| 3. | 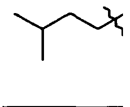 | CH₃ | 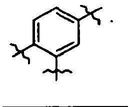 | 2 | 0 | 2 | *(R,S)* | 61-65 | Tartrate |
| 4. | 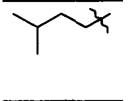 | CH₃ | 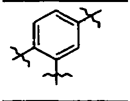 | 0 | 2 | 2 | (*R*,*S*) | 60-70 | Tartrate |
| 5. | 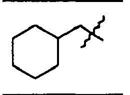 | CH₃ | 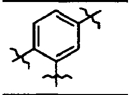 | 2 | 0 | 2 | (*R*,*S*) | 78-98 | Oxalate |
| 6. | 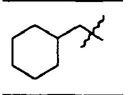 | CH₃ | 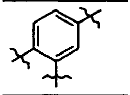 | 2 | 0 | 2 | (*R*) | 190-200 | Chlorhydrate |
| 7. | 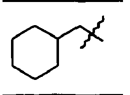 | CH₃ | 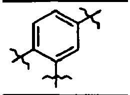 | 2 | 0 | 2 | (*S*) | 195-199 | Oxalate |
| 8. | 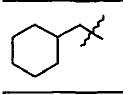 | CH₃ | 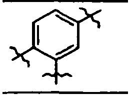 | 0 | 2 | 2 | (*R*,*S*) | 110-112 | Oxalate |
| 9. | 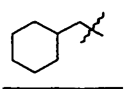 | CH₃ | 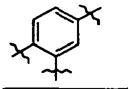 | 2 | 0 | 3 | (*R*,*S*) | 101-111 | Oxalate |
| 10 | 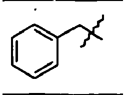 | CH₃ | 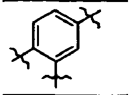 | 2 | 0 | 2 | (*R*,*S*) | 143-150 | Oxalate |
| 11 | 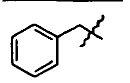 | CH₃ | 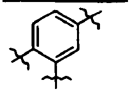 | 0 | 2 | 2 | (*R*,*S*) | 108-116 | Oxalate |
| 12 | 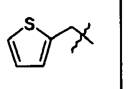 | CH₃ | 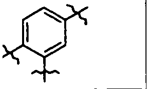 | 0 | 2 | 2 | (*R*,*S*) | 98-110 | Oxalate |
| 13 | 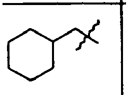 | CH₃ | 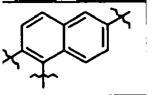 | 2 | 0 | 2 | (*R*,*S*) | 127-135 | Oxalate |
| 14 | 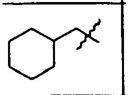 | CH₃ | 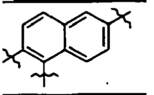 | 2 | 0 | 2 | (*R*) | 274-277 | Chlorhydrate |
| 15 | 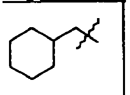 | CH₃ | 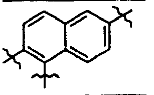 | 2 | 0 | 2 | (*S*) | 271-274 | Oxalate |
| 16 | 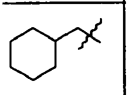 | CH₃ | 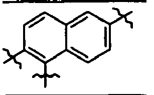 | 0 | 2 | 2 | (*R*,*S*) | 77-82 | Oxalate |
| 17 | 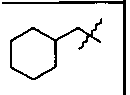 | CH₃ | 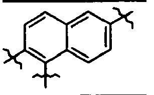 | 2 | 0 | 3 | (*R*,*S*) | 124-127 | Oxalate |
| 18 | 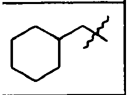 | CH₃ | 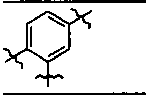 | 2 | 0 | 2 | (*S*) | 238-241 | Chlorhydrate |
| 19 | 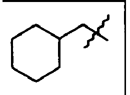 | CH₃ | 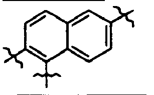 | 2 | 0 | 2 | (*S*) | 260 | Chlorhydrate |
| 2C | 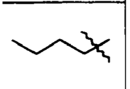 | CH₃ | 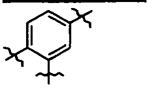 | 0 | 2 | 2 | (*R*;*S*) | 96-107 | Tartrate |
| 21 | 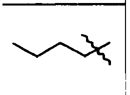 | CH₃ | 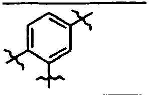 | 2 | 0 | 2 | (*R*,*S*) | 95-105 | Tartrate |
| 22 | 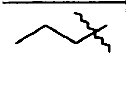 | CH₃ | 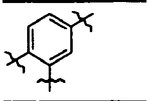 | 0 | 2 | 2 | (*R*, *S*) | 76-97 | Tartrate |
| 23 | 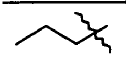 | CH₃ | 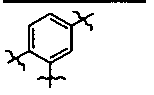 | 2 | 0 | 2 | (*R*,*S*) | 66-91 | Tartrate |

Les composés de l'invention de formule I ont fait l'objet d'essais pharmacologiques qui ont montré leur intérêt comme substances actives en thérapeutique.

Plus particulièrement, les composés de l'invention sont des antagonistes du récepteur de l'histamine du type H₃. Les récepteurs du type H₃ sont connus de l'homme du métier et leur intérêt en thérapeutique a été décrit dans la littérature («Histamine H3 receptor antagonists» Exp. Opinion Ther. Patents (2000) 10 (7) :1045-1055).

Ainsi, les composés de l'invention de formule I ont été soumis à un test d'affinité *in vitro* sur le récepteur natif de l'histamine du type H₃ dans une préparation membranaire de cerveau de rat adulte par la liaison spécifique de [³H]-N-α-méthylhistamine à ce récepteur, selon les méthodes décrites par Korte, A. et al. dans Biochem. Biophys. Res. Commun. 168, 979-986(1990) et par West, R.E. Jr. et al. dans Mol. Pharmacol. 38, 610-613(1990).

Les Kᵢ des composés de l'invention vis-à-vis des récepteurs H₃ se situent entre 0,1 nM et 5,0 µM et plus particulièrement le (2-cyclohéxylméthyl)-7-[2-(1-méthylpyrrolidin-2-yl)éthoxy]-1,2,3,4-tétrahydroisoquinoline (composé 5 du tableau) présente une Ki de 0,1 nM.

Les composés de l'invention de formule I ont été soumis aussi à un test de formation de cAMP, sur le récepteur humain de l'histamine du type H₃ transfecté dans cellules CHO, par l'inhibition de l'agonisme provoqué par la liaison spécifique de R-α-méthylhistamine à ce récepteur, selon les méthodes décrites par Lovenberg, T.W. et al. dans J. Pharmacol. Exp. Ther. 293, 771-778(2000).

Les Cl₅₀ des composés de l'invention vis-à-vis des récepteurs H₃ se situent entre 0,1 nM et 5,0 µM.

A titre d'exemple, le composé 5, inclus dans le tableau, présente une Cl₅₀<10 nM, faisant usage d'un kit EIA (Amersham) pour mesurer la formation de cAMP, sur le récepteur humain de l'histamine du type H₃ transfecté dans cellules CHO, par l'inhibition de l'agonisme provoqué par la liaison spécifique de R-α-méthylhistamine à ce récepteur.

Les composés selon l'invention ont une activité sélective du récepteur de l'histamine du type H₃. Effectivement, les composés présentent un Ki supérieur à 7,0 µM dans le test d'affinité *in vitro* sur le récepteur natif de l'histamine du type H₁ dans une préparation membranaire de cerveau de rat adulte par la liaison spécifique de [³H]-pirilamine à ce récepteur, selon la méthode décrite par Liu Y.Q. et al. dans, J.Pharmacol. Exp. Ther. 268, 959 (1994).

## Revendications

1. Composé de formule **I** 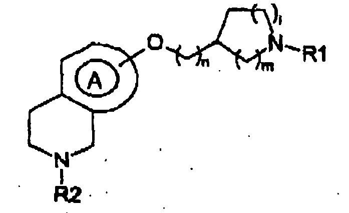 dans laquelle: 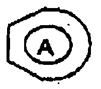 représente un carbocycle Insaturé avec doubles liaisons, éventuellement substitué par un ou deux substituants choisis, indépendamment l'un de l'autre, parmi un atome d'halogène, un hydroxy, un groupe nitro, cyano, C₁₋₂ perhalogénoalkyle, C₁₋₅ alkyle ou un phényle;
l peut prendre une valeur de 0 à 4 ;
m peut prendre une valeur de 0 à 3 ;
n peut prendre une valeur de 0 à 6 ;
-(C)I-, -(C)m- et -(C)n- représentent, indépendamment l'un de l'autre,
un groupe -C_{x-z}- alkylldène, éventuellement substitués par 1 à 4 substituants choisis parmi un atome d'halogène, un groupe hydroxy, nitro, cyano, amino, C₁₋₂ perhalogénoalkyle, C₁₋₃ alkyle ou un phényle; et de plus lorsque l, m et/ou n prend la valeur 0, -(C)₀ représente une liaison;
**R1** représente
• un atome d'hydrogène,
• un groupe C₁₋₃ alkyle,
• un C₁₋₆ alkylcarbonyle,
• un C₁₋₆ alkoxycarbonyle,
chacun pouvant être substituée, par un atome d'halogène, un groupe hydroxy, C₁₋₃ alcoxy, nitro, cyano, amino, un aryle;
• un groupe C₁₋₃ alkyle-aryle,
• un hétéroaryle monocyclique,
• un aryle; ,
les groupes aryle et hétéroaryle étant éventuellement substitué par 1 à 4 substituants choisis parmi un atome d'halogène, un groupe hydroxy, nitro, cyano, amino, C₁₋₃ monoalkylamino, C₂₋₆ dialkylamlno, C₁₋₃ alkyl, C₁₋₂ perhalogénoalkyle, C₁₋₃ halogénoalkyle, C₁₋₃ alcoxy ou un groupe C₁₋₃ alkylidénedioxy ;
R2 représente
• un groupe C₁₋₆ alkyle ou un groupe C₃₋₈ cycloalkyle chacun étant éventuellement substitués par 1 à 4 substituants choisis parmi un atome d'halogène, un groupe hydroxy, nitro, cyano, amino, C₁₋₃ monoalkylamino, C₂₋₆ dialkylamino, C₁₋₂ perhalogénoalkyle, C₁₋₃ halogénoalkyle, C₁₋₃ alcoxy, C₃₋₆ cycloalkyle, un hétéroaryle monocyclique, un hétéroaryle bicyclique, un groupe aryle lui-même éventuellement substitué par 1 à 4 substituants choisis parmi un atome d'halogène, un groupe hydroxy, nitro, cyano, amino, C₁₋₃ monoalkylamino, C₂₋₆ dialkylamino, C₁₋₃ alkyle, C₁₋₂ perhalogénoalkyle, C₁₋₃ halogénoalkyle, C₁₋₃ alcoxy ou un groupe C₁₋₃ alkylidènedioxy,
à l'état de base ou de sel d'addition à un acide, ainsi qu'à l'état d'hydrate ou de solvat.

2. Composés de formule I selon la revendication 1 dans laquelle: 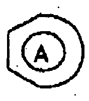 représente un carbocycle insaturé, éventuellement substitué par 1 ou 2 substituants choisis, indépendamment l'un de l'autre, parmi un atome d'halogène, un groupe hydroxy, nitro, cyano. C₁₋₂ perhatogénoalkyle ou C₁₋₃ alkyle;
l peut prendre une valeur de 1, 2 ou 3 ;
m peut prendre une valeur de 0, 1 ou 2 ;
n peut prendre une valeur de 0, 1, 2 ou 3;
-(C)l- et -(C)m- forment ensemble, avec le groupe -NR1-, un aminocycle, lié par un carbone au groupe -O-(C)n- et lorsque m prend la valeur 0, -(C)ₒ- représente une liaison;
-(C)n- représente un groupe -C₀₋₃ -alkylidène, éventuellement substitué par 1 à 4 substituants choisis parmi un atome d'halogène, un groupe hydroxy, nitro, cyano, amino, C₁₋₂ perhalogénoalkyle; et lorsque n prend la valeur 0, -(C)₀₋ représente une liaison;
R1 représente
• un atome d'hydrogène,
• un groupe C₁₋₃ alkyle,
• un C₁₋₆alkylcarbonyle,
• un C₁₋₆alkoxycarbonyle;
• C₁₋₃alkylaryle,
• un hétéroaryle,
• un groupe aryle;
les groupes aryle et hétéroaryle étant éventuellement substitué par 1 à 4 substituants choisis parmi un atome d'halogène, un groupe hydroxy, cyano, amino, C₁₋₃ monoalkylamino, C₁₋₃ alkyle, C₁₋₂ perhalogénoalkyle, C₁₋₃ halogénoalkyle, C₁₋₃ alcoxy
au C₁₋₃alkylidènedloxy,
à l'état de base ou de sel d'addition à un acide, ainsi qu'à l'état d'hydrate ou de solvat.

3. Composés de formule **I** selon la revendication 2 dans laquelle (C)l- et -(C)m- forment ensemble, avec le groupe -NR1-, un aminocycle, lié par un carbone au groupe -O-(C)n- choisi parmi azétidine, pyrrolidine, pipéridine ou azépine,
à l'état de base ou de sel d'addition à un acide, ainsi qu'à l'état d'hydrate ou de solvat.

4. Composé de formule I selon l'une des revendications 1 à 3 dans laquelle l'aminocycle dont fait partie -(C)l-, -(C)m- et-NR1- et lequel est lié par un carbone au groupe -O-(C)n-, est choisi parmi les groupes suivants: 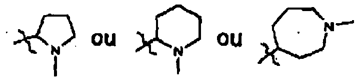 et dans laquelle
R2 représente
un groupe C₁₋₄ alkyle ou C₅₋₆ cycloalkyle chacun étant éventuellement substitués par 1 à 4 substituants choisis parmi un phényle, un hétéroaryle monocyclique un hétéroaryle bicyclique, un groupe C₃₋₆ cycloalkyle, C₁₋₂ perhalogénoalkyle, C₁₋₃ halogénoalkyle ou C₁₋₃ alcoxy; le phényle et l'hétéroaryle étant éventuellement substitués par 1 à 4 substituants choisis parmi un atome d'halogène, un groupe hydroxy, nitro, cyano, amino, C₁₋₃ monoalkylamino, C₂₋₆ dialkylamino, C₁₋₃ alkyle, C₁₋₂ perhalogénoalkyle, C₁₋₃ halogénoalkyle, C₁₋₃ alcoxy ou un groupe C₁₋₃ alkylidènedioxy.
à l'état de base ou de sel d'addition à un acide, ainsi qu'à l'état d'hydrate ou de solvat.

5. Composés de formule I selon l'une des revendications 1 à 4 dans laquelle le carbocycle insaturé avec doubles liaisons est choisi parmi un phényle ou un naphtyle,
à l'état de base ou de sel d'addition à un acide, ainsi qu'à l'état d'hydrate ou de solvat.

6. Composés de formule I selon l'une des revendications 1 à 5 dans laquelle R1 est un groupe choisi parmi un benzyloxycarbonyl, un C₁₋₃-alkyl-aryle choisi parmi benzyle ou phénéthyle, ou un hetéroaryle monocyclique choisi parmi un thiényle, furyle ou pyrrolyle ou un aryle choisi parmi un phényle ou un naphtyle,
à l'état de base ou de sel d'addition à un acide, ainsi qu'à l'état d'hydrate ou de solvat.

7. Composés de formule I selon l'une des revendications 1 à 6 dans laquelle R2 représente un groupe C₁₋₄ alkyle ou C₅₋₆ cycloalkyle chacun étant éventuellement substitués par un hétéroaryle monocyclique choisi parmi thiényle, furyle ou pyrrolyle, ou un benzotriazolyle ou un groupe aryle choisi parmi un phényle ou un naphtyle,
à l'état de base ou de sel d'additlon à un acide, ainsi qu'à l'état d'hydrate ou de solvat.

8. Composés selon l'une des revendications 1 à 7 dont les noms suivent:
1.7-{2-[1-méthylpipéridin-2-yl]éthoxy}-2-propyl-1,2,3,4-tétrahydroisoquinoline:
2. 2-Isobutyl-1-[2-(1-méthylpyrrolidln-2-yl)éthoxy]-1,2,3,4-tétrahydroisoquinoline;
3. 2-(3-méthylbutyl)-7-[2-(1-méthylpyrrolidin-2-yl)éthoxy]-1,2,3,4-tétrahydroisoquinoline;
4. 7-[(1-méthylazepan-4-yl)oxy]-2-(3-méthylbutyl)-1,2,3,4-tétrahydroisoquinoline ;
5. (2-cyclohéxylméthyl)-7-[2-(1-méthylpyrrolidin-2-yl)éthoxy]-1,2,3,4-tétrahydroisoquinoline;
6. (2-cyclohéxylméthyl)-7-{2-[(2*R*)-1-méthylpyrrolidin-2-yl]éthoxy}-1,2,3,4-tétrahydroisoquinoline;
7. (2-cyclohexylméthyl)-7-{2-[(2*S*)-1-méthylpyrrolidin-2-yl]éthoxy}-1,2,3,4-tétrahydroisoquinoline;
8. (2-cyclophéxylméthyl)-7-[(1-méthylazepan-4-yl)oxy]-1,2,3,4-tétrahydroisoquinoline;
9. (2-cyclohéxylmethyl-7-[2-(1-méthylpipéridin-2-yl)éthoxyl-1,2,3,4-tétrahydroisoquinoline;
10. 2-benzyl-7-[2-(1-méthylpyrrolidin-2-yl)éthoxy]-1,2,3,4-tétrahydroisoquinoline;
11. 2-benzyl-7-[(1-méthylazepan-4-yl)oxy]-1,2,3,4,tétrahydroisoquinoline;
12. 7-[(1-méthylazepan-4-yl)oxy]-2-(2-thiénylméthyl)-1,2,3,4-tétrahydroisoquinoline
13. (2-cyclohéxylméthyl)-8-[2-(1-méthylpyrrolidin-2-yl)éthoxyl-1,2,3,4-tétrahydrobenzo[*h*]isoquinoline;
14. (2-cyclohéxylméthyl)-8-{2-[(2*R*)-1-méthylpyrrolidin-2-yl]éthoxy}-1,2,3,4-tétrahydrobenzo[*h*]isoquinoline;
15. (2-cyclohéxylméthyl)-8-{2-[(2*S*)-1-méthylpyrrolidin-2-yl]éthoxy}-1,2,3,4-tétrahydroben[*h*]isoquinoline:
16. 2-(cyclohéxylméthyl)-8-[(1-méthylazepan-4-yl)oxy]-1,2,3,4-tetrahydrobenzo[*h*]isoquinoline ;
17. (2-cyclohéxylméthyl)-8-[2-(1-méthylpipéridin-2-yl)éthoxy]-1,2,3,4-tétrahydrobenzo[*h*]isoquinoline;
20. 2-butyl-7-[(1-méthylazepan-4-yl)oxy]-1,2,3,4-tétrahydroisoquinoline ;
21. 2-butyl-7-[2-(1-méthylpyrrolidin-2-yl)éthoxy]-1,2,3,4-tétrahydroisoquinoline ;
22. 7-[(1-méthylazepan-4-yl)oxy]-2 propyl)-1,2,3,4-tétrahydroisoquinoline;
23. 7-[2-(1-méthylpyrrolidin-2-yl)éthoxyl-2-propyl-1,2,3,4-tétrahydroisoquinoline ;
et leurs sels pharmaceutiquement acceptables.

9. Composition pharmaceutique contenant un composé de formule I, selon l'une quelconque des revendications 1 à 8, ou son sel, solvat ou hydrate, et au moins un excipient pharmaceutique.

10. Utilisation d'un composé de formule I selon l'une quelconque des revendications 1 à 8, ou son sel, solvat ou hydrate, pour la préparation d'un médicament destiné à traiter l'obésité et/ou du diabète.

11. Utilisation d'un composé de formule I selon l'une quelconque des revendications 1 à 8, ou son sel, solvat ou hydrate, pour la préparation d'un médicament destiné au traitement d'une maladie choisie parmi: des maladies du système nerveux, les troubles de la vigilance et du sommeil, la narcolepsie; la maladie d'Alzheimer et autres démences, la maladie de Parkinson, les troubles de l'attention chez l'enfant hyperkinétique, les troubles de la mémoire et de l'apprentissage, l'épilepsie, la schizophrénie, les troubles cognitifs modérés, la dépression et l'anxiété, les anxiétés de type anticipatoire, l'anxiété causée par la dépendance ou le sevrage d'alcool, de drogue, la manie, les désordres affectifs saisonniers, les migraines et les nausées.

12. Utilisation de composés de formule II 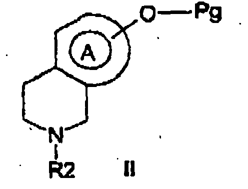 dans laquelle Pg représente un atome d'hydrogène ou un groupe protecteur et R2 et A sont tels que définis dans l'une des revendications 1 à 8 pour la préparation des composés de formule I selon l'une des revendications 1 à 8.

13. Procédé de préparation des composés de formule **I** dans laquelle R1, R2, l, m, n et le cycle A sont tels que définis dans la formule I de l'une des revendications 1 à 8, selon la réaction suivante: 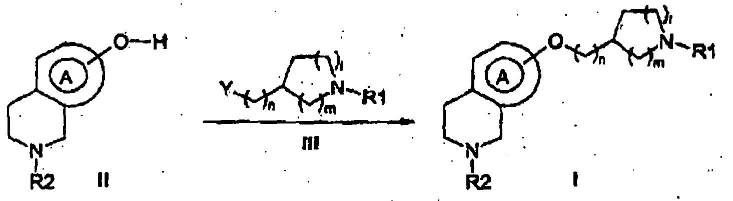 dans laquelle on effectue une substitution nucléophile, en faisant réagir un phénol de formule II, dans laquelle R2 et le cycle A sont tels que définis dans la formule I, avec une amine de formule III, dans laquelle R1, l, m et n sont définis comme dans la formule I et Y représente un atome d'halogène ou représente un «pseudo-halogène» ou bien représente un groupe hydroxy.

14. Procédé de préparation selon la revendication 13, dans lequel le composé de formule II est préparé par amino-réduction, en faisant réagir une amine secondaire de formule IV, dans laquelle R2 représente H, 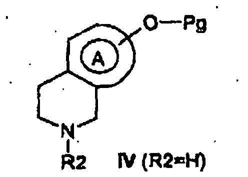 avec un aldéhyde ou une cétone de formule V (R3R4C(0)), où R3 et R4, après réaction forment ensemble R2 tel que défini dans la formule I et différent d'hydrogène, selon la formule VI: 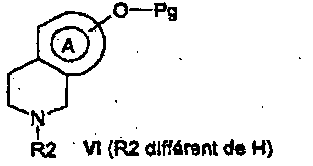 éventuellement suivi d'une déprotection.

15. Procédé de préparation selon la revendication 13, dans lequel le composé de formule II est préparé par substitution nucleophylique, en faisant réagir un composé de formule VIa, dans laquelle R2 représente un groupe benzotriazolylmethyl. 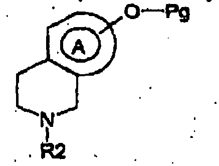 VIa (R2=benzotriazolylmethyl)
avec un réactif de Grignard de formule VII R5MgW, où W représente un atome d'halogène choisi parmi chlore, iode et brome, et R5 représente un groupe C₁₋₅ alkyle, C₁₋₂ perhalogénoalkyle, C₁₋₃ halogénoalkyle, C₃₋₆ cycloalkyle, un hétéroaryle
monocyclique ou un groupe aryle; après réaction, on forme le composé de formule VIb où R2 est égal à CH₂R₅ 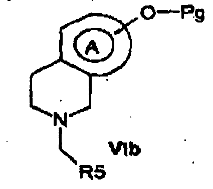 éventuellement suivi d'une déprotection.

## Claims

1. Compound of formula (I) 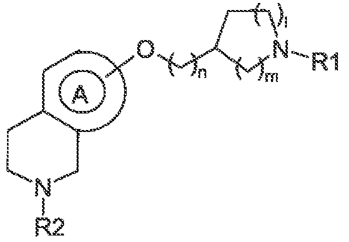 in which, 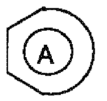 represents an unsaturated carbocycle with double bonds which is optionally substituted by one or two substituents chosen, independently of one another, from a halogen atom, a hydroxyl, a nitro, cyano, C₁₋₂ perhaloalkyl or C₁₋₃alkyl group or a phenyl;
l can take a value from 0 to 4;
m can take a value from 0 to 3;
n can take a value from 0 to 6;
-(C)ₗ-, -(C)ₘ- and -(C)ₙ- represent, independently of one another, a -C_{x-z}- alkylidene group, optionally substituted by 1 to 4 substituents chosen from a halogen atom, a hydroxyl, nitro, cyano, amino, C₁₋₂ perhaloalkyl or C₁₋₃alkyl group or a phenyl; and, furthermore, when I, m and/or n takes the value 0, -(C)ₒ₋ represents a bond;
R1 represents
• a hydrogen atom,
• a C₁₋₃ alkyl group,
• a C₁₋₆ alkylcarbonyl,
• a C₁₋₆ alkoxycarbonyl,
it being possible for each to be substituted by a halogen atom, a hydroxyl, C₁₋₃ alkoxy, nitric, cyano or amino group or an aryl;
• a C₁₋₃ alkylaryl group,
• a monocyclic heteroaryl,
• an aryl;
the aryl and heteroaryl groups optionally being substituted by 1 to 4 substituents chosen from a halogen atom, a hydroxyl, nitro, cyano, amino, C₁₋₃ monoalkylamino, C₂₋₆ dialkylamino, C₁₋₃ alkyl, C₁₋₂ perhaloalkyl, C₁₋₃ haloalkyl or C₁₋₃ alkoxy group or a C₁₋₃ alkylidenedioxy group;
R2 represents
• a C₁₋₆ alkyl group or a C₃₋₆ cycloalkyl group, each optionally being substituted by 1 to 4 substituents chosen from a halogen atom, a hydroxyl, nitro, cyano, amino, C₁₋₃ monoalkylamino, C₂₋₆ dialkylamino, C₁₋₂ perhaloalkyl, C₁₋₃ haloalkyl, C₁₋₃ alkoxy or C₃₋₆ cycloalkyl group, a monocyclic heteroaryl, a bicyclic heteroaryl or an aryl group itself optionally substituted by 1 to 4 substituents chosen from a halogen atom, a hydroxyl, nitro, cyano, amino, C₁₋₃ monoalkylamino, C₂₋₆ dialkylamino, C₁₋₃ alkyl, C₁₋₂ perhaloalkyl, C₁₋₃ haloalkyl or C₁₋₃ alkoxy group or a C₁₋₃ alkylidenedioxy group,
in the base form or in the form of an addition salt with an acid, and in the hydrate or solvate form.

2. Compound of formula (I) according to Claim 1, in which, 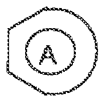 represents an unsaturated carbocycle optionally substituted by 1 or 2 substituents chosen, independently of one another, from a halogen atom or a hydroxyl, nitro, cyano, C₁₋₂ perhaloalkyl or C₁₋₃ alkyl group;
I can take a value of 1, 2 or 3;
m can take a value of 0, 1 or 2;
n can take a value of 0, 1, 2 or 3;
-(C)ₗ- and -(C)ₘ- form, together with the -NR1- group, an aminocycle bonded via a carbon to the -O-(C)ₙ- group and, when m takes the value 0, -(C)₀- represents a bond;
-(C)ₙ- represents a -C₀₋₃- alkylidene group optionally substituted by 1 to 4 substituents chosen from a halogen atom or a hydroxyl, nitro, cyano, amino or C₁₋₂ perhaloalkyl group; and, when n takes the value 0, -(C)₀- represents a bond;
R1 represents
• a hydrogen atom,
• a C₁₋₃ alkyl group,
• a C₁₋₆ alkylcarbonyl,
• a C₁₋₆ alkoxycarbonyl;
• C₁₋₃ alkylaryl,
• a heteroaryl,
• an aryl group;
the aryl and heteroaryl groups optionally being substituted by 1 to 4 substituents chosen from a halogen atom or a hydroxyl, cyano, amino, C₁₋₃ monoalkylamino, C₁₋₃ alkyl, C₁₋₂ perhaloalkyl, C₁₋₃ haloalkyl, C₁₋₃ alkoxy or C₁₋₃ alkylidenedioxy group, in the base form or in the form of an addition salt with an acid, and in the hydrate or solvate form.

3. Compound of formula (I) according to Claim 2, in which
-(C)ₗ- and -(C)ₘ- form, together with the -NR1- group, an aminocycle bonded via a carbon to the -O-(C)ₙ- group chosen from azetidine, pyrrolidine, piperidine or azepine,
in the base form or in the form of an addition salt with an acid, and in the hydrate or solvate form.

4. Compound of formula (I) according to one of Claims 1 to 3, in which the aminocycle of which -(C)ₗ-, -(C)ₘ- and -NR1- form part and which is bonded via a carbon to the -O-(C)ₙ- group is chosen from the following groups : 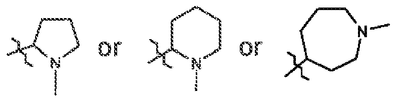 and in which
R2 represents
a C₁₋₄ alkyl or C₅₋₆ cycloalkyl group, each being optionally substituted by 1 to 4 substituents chosen from a phenyl, a monocyclic heteroaryl, a bicyclic heteroaryl or a C₃₋₆ cycloalkyl, C₁₋₂ perhaloalkyl, C₁₋₃ haloalkyl or C₁₋₃ alkoxy group; the phenyl and the heteroaryl optionally being substituted by 1 to 4 substituents chosen from a halogen atom, a hydroxyl, nitro, cyano, amino, C₁₋₃ monoalkylamino, C₂₋₆ dialkylamino, C₁₋₃ alkyl, C₁₋₂ perhaloalkyl, C₁₋₃ haloalkyl or C₁₋₃ alkoxy group or a C₁₋₃ alkylidenedioxy group,
in the base form or in the form of an addition salt with an acid, and in the hydrate or solvate form.

5. Compound of formula (I) according to one of Claims 1 to 4, in which the unsaturated carbocycle with double bonds is chosen from a phenyl or a naphthyl,
in the base form or in the form of an addition salt with an acid, and in the hydrate or solvate form.

6. Compound of formula (I) according to one of Claims 1 to 5, in which R1 is a group chosen from a benzyloxycarbonyl, a C₁₋₃ alkylaryl chosen from benzyl or phenethyl, a monocyclic heteroaryl chosen from a thienyl, furyl or pyrrolyl or an aryl chosen from a phenyl or a naphthyl,
in the base form or in the form of an addition salt with an acid, and in the hydrate or solvate form.

7. Compound of formula (I) according to one of Claims 1 to 6, in which R2 represents a C₁₋₄ alkyl or C₅₋₆ cycloalkyl group, each optionally being substituted by a monocyclic heteroaryl chosen from thienyl, furyl or pyrrolyl or a benzotriazolyl or an aryl group chosen from a phenyl or a naphthyl,
in the base form or in the form of an addition salt with an acid, and in the hydrate or solvate form.

8. Compound according to one of Claims 1 to 7, the names of which follow
1. 7-{2-[1-methylpiperidin-2-yl]ethoxy}-2-propyl-1,2,3,4-tetrahydroisoquinoline;
2. 2-isobutyl-7-[2-(1-methylpyrrolidin-2-yl)ethoxy]-1,2,3,4-tetrahydroisoquinoline;
3. 2-(3-methylbutyl)-7-[2-(1-methylpyrrolidin-2-yl)ethoxy]-1,2,3,4-tetrahydroisoquinoline;
4. 7-[(1-methylazepan-4-yl)oxy]-2-(3-methylbutyl)-1,2,3,4-tetrahydroquinoline;
5. 2-(cyclohexylmethyl)-7-[2-(1-methylpyrrolidin-2-yl)ethoxy]-1,2,3,4-tetrahydroisoquinoline;
6. 2-(cyclohexylmethyl)-7-{2-[(2*R*)-1-methylpyrrolidin-2-yl]ethoxy}-1,2,3,4-tetrahydroisoquinoline;
7. 2-(cyclohexylmethyl)-7-{2-[(2*S*)-1-methylpyrrolidin-2-yl]ethoxy}-1,2,3,4-tetrahydroisoquinoline;
8. 2-(cyclohexylmethyl)-7-[(1-methylazepan-4-yl)oxy]-1,2,3,4-tetrahydroisoquinoline;
9. 2-(cyclohexylmethyl)-7-[2-(-methylpiperidin-2-yl)ethoxy]-1,2,3,4-tetrahydroisoquinoline;
10. 2-benzyl-7-[2-(1-methylpyrrolidin-2-yl)ethoxy]-1,2,3,4-tetrahydroisoqujnoline;
11. 2-benzyl-7-[(1-methylazepan-4-yl)oxy]-1,2,3,4-tetrahydroisoquinoline;
12. 7-[(1-methylazepan-4-yl)oxy]-2-(2-thienylmethyl)-1,2,3,4-tetrahydroisoquinoline;
13. 2-(cyclohexylmethyl)-8-[2-(1-methylpyrrolidin-2-yl)ethoxy]-1,2,3,4-tetrahydrobenzo[*h*]isoquinoline;
14. 2-(cyclohexylmethyl)-8-{2-[(2R)-1-methylpyrrolidin-2-yl]ethoxy}-1,2,3,4-tetrahydrobenzo[*h*]isoquinoline;
15. 2-(cyclohexylmethyl)-8-{2-[{2S}-1-methylpyrrolidin-2-yl]ethoxy}-1,2,3,4-tetrahydrobenzo[*h*]isoquinoline;
16. 2-(cyclohexylmethyl)-8-[(1-methylazepan-4-yl)oxy]-1,2,3,4-tetrahydrobenzo[*h*]isoquinoline;
17. 2-(cyclohexylmethyl)-8-[2-(1-methylpiperidin-2-yl)ethoxy]-1,2,3,4-tetrahydrobenzo[*h*]isoquinoline;
20. 2-butyl-7-[(1-methylazepan-4-yl)oxy]-1,2,3,4-tetrahydroisoquinoline;
21. 2-butyl-7-[2-(1-methylpyrrolidin-2-yl)ethoxy]-1,2,3,4-tetrahydroisoquinoline;
22. 7-[(1-methylazepan-4-yl)oxy]-2-propyl-1,2,3,4-tetrahydroisoquinoline;
23. 7-[2-(1-methylpyrrolidin-2-yl)ethoxy]-2-propyl-1,2,3,4-tetrahydroisoquinoline;
and their pharmaceutically acceptable salts.

9. Pharmaceutical composition comprising a compound of formula (I) according to any one of Claims 1 to 8, or its salt, solvate or hydrate, and at least one pharmaceutical excipients

10. Use of a compound of formula (1) according to any one of Claims 1 to 8, or its salt, solvate or hydrate, in the preparation of a medicament intended to treat obesity and/or diabetes.

11. Use of a compound of formula (I) according to any one of Claims 1 to 8, or its salt, solvate or hydrate, in the preparation of a medicament intended for the treatment of a disease chosen from: diseases of the nervous system, watchfulness and sleep disorders, narcolepsy, Alzheimer's disease and other types of dementia, Parkinson's disease, attention disorders in hyperkinetic children, memory and learning disorders, epilepsy, schizophrenia, moderate cognitive disorders, depression, anxiety, anticipatory anxiety, anxiety caused by dependence on or weaning from alcohol or drugs, mania, seasonal affective disorder, migraine and nausea.

12. Use of the compound of formula (II) 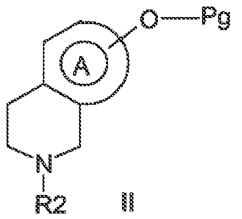 in which Pg represents a hydrogen atom or a protective group and R2 and A are as defined in one of Claims 1 to 8, in the preparation of the compound of formula (I) according to one of Claims 1 to 8.

13. Process for the preparation of the compound of formula (I) in which R1, R2, l, m, n and the A ring are as defined in the formula (I) of one of Claims 1 to 8, according to the following reaction: 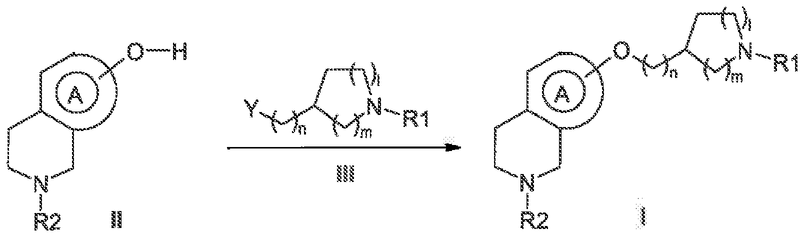 in which a nucleophilic substitution is carried out by reacting a phenol of formula (II), in which R2 and the A ring are as defined in the formula (I), with an amine of formula (III), in which R1, I, m and n are defined as in the formula (I) and Y represents a halogen atom or represents a " pseudohalogen " or else represents a hydroxyl group.

14. Preparation process according to Claim 13, in which the compound of formula (II) is prepared by reductive amination by reacting a secondary amine of formula (IV), in which R2 represents H, 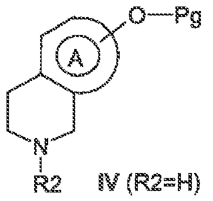 with an aldehyde or a ketone of formula (V) (R3R4C(O)), where R3 and R4, after reaction, together form R2 as defined in the formula (I) and other than hydrogen, according to the formula (VI): 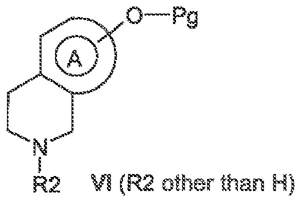 optionally followed by deprotection.

15. Preparation process according to Claim 13, in which the compound of formula (II) is prepared by nucleophilic substitution by reacting a compound of formula (VIa), in which R2 represents a benzotriazolylmethyl group, 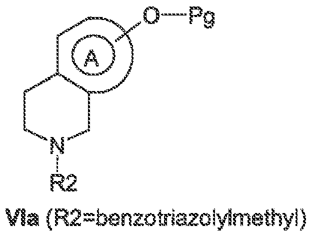 with a Grignard reagent of formula (VII) R5MgW, where W represents a halogen atom chosen from chlorine, iodine and bromine and R5 represents a C₁₋₅ alkyl, C₁₋₂ perhaloalkyl, C₁₋₃ haloalkyl or C₃₋₆ cycloalkyl group, a monocyclic heteroaryl or an aryl group; after reaction, the compound of formula (VIb) where R2 is equal to CH₂R₅ is formed, 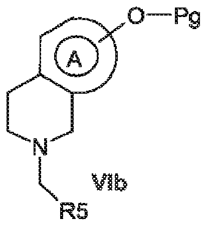 optionally followed by deprotection.

## Patentansprüche

1. Verbindung der Formel I 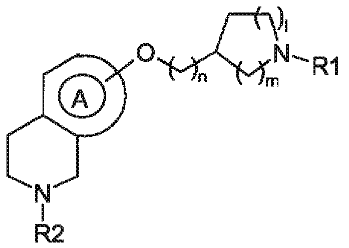 mit den folgenden Bedeutungen: 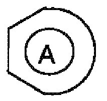 bedeutet einen ungesättigten Carbocyclus mit Doppelbindungen, der gegebenenfalls durch einen oder zwei Substituenten substituiert ist, die unabhängig voneinander aus der Reihe Halogenatom, Hydroxy, eine Nitro-, Cyano-, Perhalogen-C₁₋₂-alkyl-, C₁₋₃-Alkyl- oder Phenylgruppe ausgewählt sind;
1. einen Wert von 0 bis 4 annehmen kann;
m einen Wert von 0 bis 3 annehmen kann;
n einen Wert von 0 bis 6 annehmen kann;
-(C)l-, -(C)m- und -(C)n- unabhängig voneinander eine -C_{x-z}-Alkylidengruppe bedeuten, die gegebenenfalls durch 1 bis 4 Substituenten substituiert ist, die aus der Reihe Halogenatom, Hydroxy-, Nitro-, Cyano-, Amin-, Perhalogen-C₁₋₂-alkyl-, C₁₋₃-Alkyl- oder Phenylgruppe ausgewählt sind; und weiterhin, wenn l, m und/oder n den Wert 0 annehmen, -(C)₀ eine Bindung bedeutet;
R1 bedeutet
• ein Wasserstoffatom,
• eine C₁₋₃-Alkylgruppe,
• eine C₁₋₆-Alkylcarbonylgruppe,
• eine C₁₋₆-Alkoxycarbonylgruppe,
die jeweils durch ein Halogenatom, eine Hydroxy-, C₁₋₃-Alkoxy-, Nitro-, Cyano-, Amino-, Arylgruppe substituiert sein können;
• eine C₁₋₃-Alkylarylgruppe,
• einen monocyclischen Heterocyclus,
• ein Aryl;
wobei die Aryl- und Heteroarylgruppen gegebenenfalls durch 1 bis 4 Substituenten substituiert sein können, die aus der Reihe Halogenatom, Hydroxy-, Nitro-, Cyano-, Amino- C₁₋₃-Monoalkylamzno-, C₂₋₆-Dialkylamino-, C₁₋₃-Alkyl-, Perhalogen-C₁₋₂-alkyl-, Halogen-C₁₋₃-alkyl-, C₁₋₃-Alkoxy- oder C₁₋₃-Alkylidendioxygruppe ausgewählt sind;
R2 bedeutet
• eine C₁₋₆-Alkylgruppe oder eine C₃₋₆-Cycloalkylgruppe, die jeweils gegebenenfalls durch 1 bis 4 Substituenten substituiert sind, die aus der Reihe Halogenatom, Hydroxy-, Nitro-, Cyano-Amino-, C₁₋₃-Monoalkylamino-, C₂₋₆-Dialkylamino-Perhalogen-C₁₋₂-alkyl-, Halogen-C₁₋₃-alkyl-, C₁₋₃-Alkoxy-, C₃₋₆-Cycloalkylgruppe, monocyclisches Heteroaryl, bicyclisches Heteroaryl, Arylgruppe, die selbst gegebenenfalls mit 1 bis 4 Substituenten substituiert ist, die aus der Reihe Halogenatom, Hydroxy-, Nitro-, Cyano-, Amino-, C₁-₃-Monoalkylamino-, C₂₋₆-Dialkylamino-, C₁₋₃-Alkyl-, Perhalogen-C₁₋₂-Alkyl-, Halogen-C₁₋₃-alkyl-, C₁₋₃-Alkoxy- oder C₁₋₃-Alkylidendioxygruppe ausgewählt sind, ausgewählt sind,
als Base oder als Säureadditionssalz sowie als Hydrat oder als Solvat.

2. Verbindungen der Formel I nach Anspruch 1, mit den folgenden Bedeutungen: 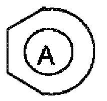 bedeutet einen ungesättigten Carbocyclus, der gegebenenfalls durch 1 oder 2 Substituenten substituiert ist, die unabhängig voneinander aus der Reihe Halogenatom, Hydroxy-, Nitro-, Cyano-, Perhalogen-C₁₋₂-alkyl- oder C₁₋₃-Alkylgruppe ausgewählt sind;
l kann einen Wert von 1, 2 oder 3 annehmen;
m kann einen Wert von 0, 1 oder 2 annehmen;
n kann einen Wert von 0, 1, 2 oder 3 annehmen;
-(C)l- und -(C)m- bilden zusammen mit der -NR₁-Gruppe einen Aminocyclus, der an die -O-(C)n-Gruppe über einen Kohlenstoff gebunden ist und, wenn m den Wert 0 annimmt, -(C)₀- eine Bindung bedeutet;
-(C)n- bedeutet eine -C₀₋₃-Alkylidengruppe, die gegebenenfalls durch 1 bis 4 Substituenten substituiert ist, die aus der Reihe Halogenatom, Hydroxy-, Nitro-, Cyano-, Amin-, Perhalogen-C₁₋₂-alkylgruppe ausgewählt sind; und wenn n den Wert 0 annimmt, bedeutet (C)₀-eine Bindung;
R1 bedeutet
• ein Wasserstoffatom,
• eine C₁₋₃-Alkylgruppe,
• eine C₁₋₆-Alkylcarbonylgruppe,
• eine C₁₋₆-Alkoxycarbonylgruppe;
• C₁₋₃-Alkylaryl,
• ein Heteroaryl,
• eine Arylgruppe;
wobei die Aryl- und Heteroarylgruppen gegebenenfalls durch 1 bis 4 Substituenten substituiert sein können, die aus der Reihe Halogenatom, Hydroxy-, Cyano-, Amino-, C₁₋₃-Monoalkylamino-, C₁₋₃-Alkyl-, Perhalogen-C₁₋₂-alkyl-, Halogen-C₁₋₃-alkyl-, C₁₋₃-Alkoxy- oder C₁₋₃-Alkylidendioxy ausgewählt sind,
als Base oder als Säureadditionssalz sowie als Hydrat oder Solvat.

3. Verbindungen der Formel I nach Anspruch 2 mit den folgenden Bedeutungen:
(C)l- und -(C)m- bilden mit der -NR1-Gruppe einen Aminocyclus, der an die -O-(C)n-Gruppe über einen Kohlenstoff gebunden ist, ausgewählt aus der Reihe Azetidin, Pyrrolidin, Piperidin oder Azepin,
als Base oder als Säureadditionssalz sowie als Hydrat oder Solvat.

4. Verbindung der Formel I nach einem der Ansprüche 1. bis 3, in der der Aminocyclus, zu dem -(C)l-, -(C)mund -NR1- gehört und der an die -O-(C)n-Gruppe über einen Kohlenstoff gebunden ist, aus den folgenden Gruppen ausgewählt ist: 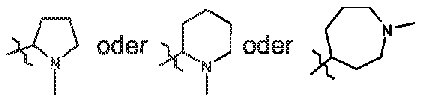 und in der
R2 eine C₁₋₄-Alkylgruppe oder C₅₋₆-Cycloalkylgruppe, die gegebenenfalls durch 1 bis 4 Substituenten substituiert sind, die aus der Reihe Phenyl, monocyclisches Heteroaryl, bicyclisches Heteroaryl, C₃₋₆-Cycloalkylgruppe, Perhalogen-C₁₋₂-alkyl, Halogen-C₁₋₃-alkyl oder C₁₋₃-Alkoxy ausgewählt sind, bedeutet; wobei Phenyl und Heteroaryl gegebenenfalls durch 1 bis 4 Substituenten substituiert sind, die aus der Reihe Halogenatom, Hydroxy-, Nitro-, Cyano-, Amino-, C₁₋₃-Monoalkylamino-, C₂₋₆-Dialkylamino-, C₁₋₃-Alkyl-, Perhalogen-C₁₋₂-alkyl-, Halogen-C₁₋₃-alkyl, C₁₋₃-Alkoxy-oder C₁₋₃-Alkylidendioxygruppe ausgewählt sind,
als Base oder als Säureadditionssalz sowie als Hydrat oder Solvat.

5. Verbindungen der Formel 1 nach einem der Ansprüche 1 bis 4, in der der ungesättigte Carbocyclus mit Doppelbindungen aus der Reihe Phenyl oder Naphthyl ausgewählt ist,
als Base oder als Säureadditionssalz sowie als Hydrat oder Solvat.

6. Verbindungen der Formel I nach einem der Ansprüche 1 bis 5, in der es sich bei R1 um eine Gruppe, ausgewählt aus Benzyloxycarbonyl, C₁₋₃-Alkylaryl, ausgewählt aus Benzyl oder Phenethyl, oder monocyclisches Heteroaryl, ausgewählt aus der Reihe Thienyl, Furyl oder Pyrrolyl oder Aryl, ausgewählt aus der Reihe Phenyl oder Naphthyl, handelt,
als Base oder als Säureadditionssalz sowie als Hydrat oder Solvat.

7. Verbindungen der Formyl 1 nach einem der Ansprüche 1 bis 6, in der R2 eine C₁₋₄-Alkyl- oder C₅₋₆-Cycloalkylgruppe bedeutet, die jeweils gegebenenfalls durch ein monocyclisches Heteroaryl substituiert sind, das aus der Reihe Thienyl, Furyl oder Pyrrolyl ausgewählt ist, oder Benzotriazolyl oder eine Arylgruppe, ausgewählt aus der Reihe Phenyl oder Naphthyl,
als Base oder als Säureadditionssalz sowie als Hydrat oder Solvat.

8. Verbindungen nach einem der Ansprüche 1 bis 7 mit den folgenden Bezeichnungen:
1. 7-(2-[1-Methylpiperidin-2-yl]ethoxy)-2-propyl-1,2,3,4-tetrahydroisochinolin;
2. 2-Isobutyl-7-[2-(1-methylpyrrolidin-2-yl)ethoxy]-1,2,3,4-tetrahydroisochinolin;
3. 2-(3-Methylbutyl)-7-[2-(1-methylpyrrolidin-2-yl)ethoxyl-1,2,3,4-tetrahydroisochinolin;
4. 7-[(1-Methylazepan-4-yl)oxy]-2-(3-methylbutyl)-1,2,3,4-tetrahydroisochinolin;
5. (2-Cyclohexylmethyl)-7-[2-(1-methylpyrrolidin-2-yl)ethoxy]-1,2,3,4-tetrahydroisochinolin;
6. (2-Cyclohexylmethyl)-7-(2-[(2R)-1-methylpyrrolidin-2-yl]ethoxy)-1,2,3,4-tetrahydroisochinolin;
7. (2-Cyclohexylmethyl)-7-(2-[(25)-1-methylpyrrolidin-2-yl]ethoxy)-1,2,3,4-tetrahydroisochinolin;
8. (2-Cyclohexylmethyl)-7-[(1-methylazepan-4-yl)oxy]-1,2,3,4-tetrahydroisochinolin;
9. (2-Cyclohexylmethyl)-7-[2-(1-methylpiperidin-2-yl)ethoxy]-1,2,3,4-tetrahydroisochinolin;
10. 2-Benzyl-7-[2-(l-methylpyrrolidin-2-yl)ethoxy]-1,2,3,4-tetrahydroisochinolin;
11. 2-Benzyl-7-[(1-methylazepan-4-yl)oxy]-1,2,3,4-tetrahydroisochinolin;
12. 7-[(1-Methylazepan-4-yl)oxy]-2-(2-thienylmethyl)-1,2,3,4-tetrahydroisochinolin;
13. (2-Cyclohexylmethyl)-8-[2-(1-methylpyrrolidin-2-yl)ethoxy]-1,2,3,4-tetrahydrobenzo[*h*]isochinolin;
14. (2-Cyclohexylmethyl)-8-[2-[(2*R*)-1-methylpyrrolidin-2-yl]ethoxy]-1,2,3,4-tetrahydrobenzo[*h*]isochinolin;
15. (2-Cyclohexylmethyl)-8-(2-[(2*S*)-1-methylpyrrolidin-2-yl]ethoxy)-1,2,3, tetrahydrobenzo[*h*]isochinolin;
16. 2-(Cyclohexylmethyl)-8-[(1-methylazepan-4-yl)oxy]-1,2,3,4-tetrahydrobenzo[*h*]isochinolin;
17. (2-Cyclohexylmethyl)-8-[2-(1-methylpiperidin-2-yl)ethoxy]-1,2,3,4-tetrahydrobenzo[*h*]isochinolin;
20. 2-Butyl-7-[(1-methylazepan-4-yl)oxy]-1,2,3,4-tetrahydroisochinolin;
21. 2-Butyl-7-[2-(1-methylpyrrolidin-2-yl)ethoxy]-1,2,3,4-tetrahydroisochinolin;
22. 7-[(1-Methylazepan-4-yl)oxy]-2-propyl-1,2,3,4-tetrahydroisochinolin;
23. 7-[2-(1-methylpyrrolidin-2-yl)ethoxy]-2-propyl-1,2,3,4-tetrahydroisochinolin;
und ihre pharmazeurisch unbedenklichen Salze.

9. Pharmazeutische Zusammensetzung, enthaltend eine Verbindung der Formel I nach einem der Ansprüche 1 bis 8 oder ein Salz, Solvat oder Hydrat der Verbindung sowie mindestens einen pharmazeutischen Grundstoff.

10. Verwendung einer Verbindung der Formel I nach einem der Ansprüche 1 bis 8 oder eines Salzes, Solvats oder Hydrats der Verbindung zur Herstellung eines Arzneimittels für die Behandlung von Adipositas und/oder Diabetes.

11. Verwendung einer Verbindung der Formel I nach einem der Ansprüche 1 bis 8 oder eines Salzes, Solvats oder Hydrats der Verbindung zur Herstellung eines Arzneimittels für die Behandlung von einer Krankheit, ausgewählt aus der Reihe: Krankheiten des Nervensystems, Wachhaftigkeits- und Schlafstörungen, Narkolepsie; Morbus Alzheimer und andere Arten von Demenz, Morbus Parkinson, hyperkinetisches Syndrom des Kindesalters, Gedächtnis- und Lernstörungen, Epilepsie, Schizophrenie, moderate kognitive Störungen, Depression und Angstgefühl, Erwartungsangst, Dependenzangst bzw.
Alkohol- oder Drogenentwöhnungsangst, Manie, saisonal affektive Störungen, Migräne und Übelkeit.

12. Verwendung von Verbindungen der Formel II, 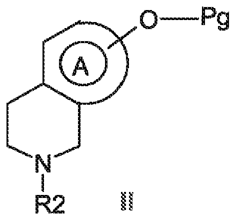 in der Pg ein Wasserstoffatom oder eine Schutzgruppe bedeutet und R2 und A wie in einem der Ansprüche 1 bis 8 definiert sind, zur Herstellung von Verbindungen der Formel I nach einem der Ansprüche bis 8,

13. verfahren zur Herstellung von Verbindungen der Formel I, in der R1, R2, 1, m, n und der Ring A wie in Formel 1 nach einem der Ansprüche 1 bis 8 definiert sind, gemäß der folgenden Reaktion: 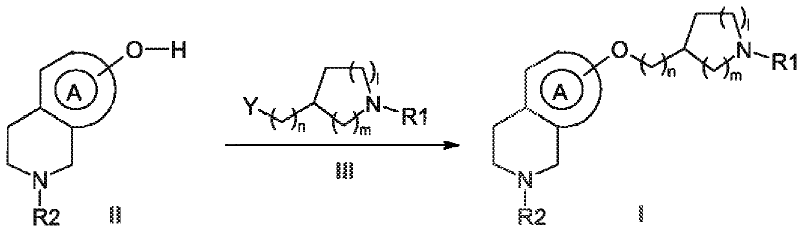 bei der man durch Umsetzen eines Phenols der Formel II, in der R2 und der Ring A wie in Formel I definiert sind, mit einem Amin der Formel III, in der R1, l, m und n wie in Formel I definiert sind und Y ein Halogenatom oder ein "Pseudohalogen" oder eine Hydroxygruppe bedeutet, eine nukleophile Substitution durchführt.

14. Herstellungsverfahren nach Anspruch 13, wobei die Verbindung II durch Amino-Reduktion hergestellt wird, und zwar dadurch, dass man ein sekundäres Amin der Formel IV, in der R2 H bedeutet, 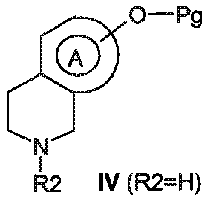 mit einem Aldehyd oder einem Keton der Formel V (R3R4C (O)), wo R3 und R4 nach Umsetzen gemeinsam R2 wie in Formel 1 definiert bilden und nicht Wasserstoff sind, gemäß der Formel VI: 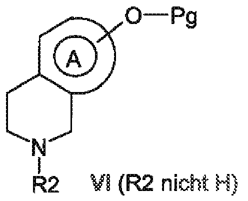 mittels Amino-Reduktion umsetzt, wonach gegebenenfalls entschützt wird.

15. Herstellungsverfahren nach Anspruch 13, wobei die Verbindung II durch nukleophile Substitution hergestellt wird, und zwar dadurch, dass man eine Verbindung der Formel VIa, in der R2 eine Benzotriazolylmethylgruppe bedeutet, 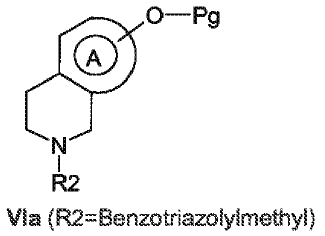 mit einem Grignard-Reagens der Formel VII R5MgW, wobei W ein Halogenatom, ausgewählt aus der Reihe Chlor, Iod und Brom bedeutet und R5 eine C₁₋₅-Alkyl- Perhalogen-C₁-₂-alkyl-, Halogen-C₁₋₃-alkyl-, C₃₋₆-cycloalkyl-, monocyclische Heteroaryl- oder Arylgruppe, bedeutet; nach dem Umsetzen bildet man die Verbindung der Formel VIb, in der R2 CH₂R₅ bedeutet, 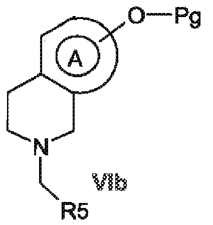 umsetzt, wonach gegebenenfalls entschützt wird.
